**Europäisches Patentamt**

**European Patent Office**

**Office européen des brevets**

(19)

(11) Veröffentlichungsnummer: **0 236 785**
**B1**

(12) **EUROPÄISCHE PATENTSCHRIFT**

(45) Veröffentlichungstag der Patentschrift:
**05.04.89**

(51) Int. Cl.⁴: **C07C 39/10, C07C 37/60**

(21) Anmeldenummer: **87102100.2**

(22) Anmeldetag: **14.02.87**

(54) Verfahren zur Herstellung von Trihydroxybenzolen.

(30) Priorität: **11.03.86 DE 3607924**

(43) Veröffentlichungstag der Anmeldung:
**16.09.87 Patentblatt 87/38**

(45) Bekanntmachung des Hinweises auf die Patenterteilung:
**05.04.89 Patentblatt 89/14**

(84) Benannte Vertragsstaaten:
**AT CH DE FR GB GR IT LI NL SE**

(56) Entgegenhaltungen:
**EP-A- 0 122 374**
**DE-A- 2 138 735**
**US-A- 3 959 388**
**US-A- 4 387 252**

**PATENT ABSTRACTS OF JAPAN, unexamined
applications, C Feld, Band 4, Nr. 106, 30. Juli 1980, THE
PATENT OFFICE JAPANESE GOVERNMENT, p. 149 C 20**

(73) Patentinhaber: **Degussa Aktiengesellschaft,
Weissfrauenstrasse 9, D-6000 Frankfurt am Main 1(DE)**

(72) Erfinder: **Prescher, Günter, Dr., Liesingstrasse 2,
D-6450 Hanau 9(DE)**
Erfinder: **Ritter, Gebhard, Dr., Lucasweg 1,
D-6100 Darmstadt(DE)**
Erfinder: **Sauerstein, Holger, Goethestrasse 1,
D-6451 Grosskrotzenburg(DE)**

**Beschreibung**

Die Erfindung betrifft die Herstellung von 1,2,3-Trihydroxybenzol (Pyrogallol) und 1,2,4-Trihydroxybenzol (Hydroxyhydrochinon).

Beide Substanzen sind als starke Reduktionsmittel bekannt und werden dementsprechend technisch eingesetzt:

Pyrogallol wird in der Fotografie und Lithographie, in der Galvanotechnik und für kosmetische Zubereitungen sowie für gewisse Insektizide verwendet; Hydroxyhydrochinon wird als Stabilisator, Antioxydans und Polymerisationsinhibitor, sowie wegen seiner gegenüber Pyrogallol geringeren Toxizität auch in der kosmetischen Industrie benutzt. (ULLMANN, 4. Auflage, Band 18, Seite 223).

Die wichtigste industrielle Gewinnungsmethode für Pyrogallol war bisher die Decarboxylierung der natürlich vorkommenden Gallussäure, deren Menge aber begrenzt ist, und die selbst recht teuer ist.

Es bestand daher schon lange ein Bedürfnis, Pyrogallol auch synthetisch herstellen zu können. Schon seit fast 100 Jahren beschäftigte man sich mit einer Umsetzung zwischen Resorcin und Wasserstoffperoxid, ohne zu einem Erfolg zu kommen.

Wurster erhielt im Jahre 1887 überhaupt kein Produkt, sondern gab nur an, daß Resorcin durch Wasserstoffperoxid rasch nahezu zerstört würde (B, 20, 1887, Seite 2938). H. v. Liebig beschrieb dann im Jahre 1912 das Auftreten eines dunkelbraunen Lackes mit der Formel $C_{18}H_{14}O_8$, dem er den Namen "Resorcinbraun" gab.

Er nahm an, daß es dem aus Resorcin, Wasserstoffperoxid und Ammoniak anfallenden Resorcinblau oder Lackmoid entsprechend auch polymolekular sei. (J. Pr. [2] 85, Seite 258). R. Hettinger bewies dann aber, daß das Produkt Lackmoid nicht einheitlich sei (Biochem. Z. 65, Seite 177 (1914)).

Da die Reaktion ohne Katalysator nicht zu einem definierten Produkt führte, wurde sie in Gegenwart von Katalysatoren, wie z.B. Ferrosulfat, durchgeführt und dabei nur eine Lumineszenz festgestellt; weitere Angaben fehlten (Z. anorg. Ch. 199, Seite 400/403, 1931).

Bei Verwendung von Wolframsäure als Katalysator entstand Maleinsäure und Kohlendioxid (J. Indian Chem. Soc. 19, (1942) Seite 499), und in Gegenwart von wäßrigem Natriumhydroxid wurde mit Kaliumperoxidisulfat nur Kohlendioxid erhalten (Atti X Congr. int. Chim. Rom, 1938, Bd. 3, Seite 682 ff.).

1963 nahm Musso bei der Untersuchung der Autoxydationsprodukte von Resorcin in ammoniakalischer Lösung, die er auch mit Wasserstoffperoxid durchführte, kondensierte Verbindungen (Phenoxazone) als Endprodukte an (B, 96, Seite 1579 ff. (1963)).

Bei einer späteren, kinetischen Untersuchung der Reaktion zwischen Resorcin und Wasserstoffperoxid in Gegenwart von Cu-II-Ionen und pH-Werten von 10,7 bis 12,7 wurde als Endprodukt versuchsweise ein Dihydroxytrichinon angegeben. Ew wurde außerdem festgestellt, daß bei einem pH-Wert von nur 9 die Reaktion außerordentlich langsam ablief (Inorg. Chim. Acta, Bd. 99, 1985, Seite 217 ff.). Der Versuch, die Hydroxylierung von Resorcin und Wasserstoffperoxid mit den hochgiftigen Fluorketonen als Katalysatoren vorzunehmen, führte zwar zu einer sehr geringen Bildung von Trihydroxybenzolen, aber mindestens 90% des umgesetzten Wasserstoffperoxids ließen sich trotz dieser Katalysatoren nicht zu den gewünschten Produkten umsetzen (JP-PS 50 151 832 [1975]).

Nach diesen zwischen 1887 und 1985 erfolgten Veröffentlichungen über die Reaktion zwischen Resorcin und Wasserstoffperoxid mußte die Fachwelt daher annehmen, daß hierbei zahlreiche, zum größten Teil nicht eindeutig festgelegte Verbindungen entstanden, und daß diese Reaktion zur Herstellung von Pyrogallol und Hydroxyhydrochinon nicht geeignet sei, und schon gar nicht im technischen Maße.

Die Fachwelt ging daher auch völlig andere Wege, um Pyrogallol herzustellen, z.B. durch Erhitzen von bestimmten Diaminophenolen unter sauren Bedingungen (DE-OS 2 445 336), durch Dealkylieren von aufwendig über mehrfach halogenierte aromatische Äther hergestellte Pyrogalloläther (DE-OS 2 627 874), über Tetrahalogenderivate des Cyclohexanons und deren basisch katalysierten Hydrolyse (DE-OS 2 653 446). Auch die Oxydation eines ein- oder zweiwertigen aromatischen Hydroxyaldehyds, der zunächst speziell hergestellt werden mußte, unter basischen Bedingungen mit u.a. Wasserstoffperoxid wurde beschrieben (EP-PS 0 025 659).

Trotz des Aufwandes dieser zum Teil mehrstufigen Verfahren ließen die Ausbeuten an Pyrogallol und Hydroxyhydrochinon erheblich zu wünschen übrig, und keines dieser Verfahren war technisch zur Herstellung der beiden Stoffe geeignet.

Aufgabe der Erfindung ist es nun, Pyrogallol und Hydroxyhydrochinon ohne Aufwand durch Oxydation von Resorcin mit Wasserstoffperoxid herzustellen.

Es wurde nun gefunden, daß sich diese Aufgabe lösen läßt, wenn man Resorcin bei mindestens 60°C mit wäßrigem Wasserstoffperoxid in Abwesenheit eines Katalysators in Kontakt bringt, wobei die anfängliche Wassermenge des Gemisches vor Beginn der Reaktion zwischen 0,1 bis 36 Gewichtsprozent, bezogen auf das Gemisch, liegt.

Günstig ist es, Resorcin bei mindestens 80°C mit dem wäßrigen Wasserstoffperoxid zu kontaktieren. Selbst Temperaturen bis herauf zu 150°C können von Anfang an verwendet werden. Bei Verwendung noch höherer Temperatur muß jedoch zusätzlich geheizt werden. Als besonders günstige Anfangstemperaturen bei dem Inkontaktbringen von Resorcin und wäßrigem Wasserstoffperoxid haben sich der Bereich zwischen 110 und 120°C erwiesen. Die erfindungsgemäß anzuwendenden höheren Temperaturen haben anscheinend die Umsetzung in Richtung der Bildung von Trihydroxybenzolen gelenkt. Aber auch die vor Beginn der Reaktion vorliegenden Wassermengen haben einen Einfluß auf die Reaktion.

Bevorzugte Wassermengen vor Beginn der Reaktion liegen bei 0,5 bis 15 Gewichtsprozent, bezogen auf das Gemisch aus Resorcin und Wasserstoffperoxid, besonders bevorzugt sind Wassermengen von 0,8 bis 10 Gewichtsprozent, ganz besonders bevorzugt von 0,8 bis 6 Gewichtsprozent.

Das Einsetzen einer Resorcinschmelze war hiernach besonders erfolgreich. Auch in dem genannten Temperaturbereich gesättigte wäßrige Lösungen von Resorcin, z.B. gesättigt bei 80 oder 100°C, konnten verwendet werden. Die Reaktionszeiten steigen jedoch im allgemeinen mit steigendem Wassergehalt an, siehe z.B. Beispiel 2 und 6.

Auch die Ausbeuten werden durch den Wassergehalt beeinflußt. Dieser Einfluß ist zwar bei Einsetzen einer Schmelze von Resorcin nicht ins Gewicht fallend, siehe Beispiel 2 bis 4, tritt jedoch bei Verwendung einer gesättigten Lösung deutlich in Richtung einer Abnahme der Ausbeute auf, siehe Beispiel 6 und 7.

In diesen genannten Fällen wurde das gleiche Molverhältnis von Resorcin zu Wasserstoffperoxid (100%ig) eingesetzt, nämlich 10:1. Dieses Verhältnis liegt normalerweise bei 5 bis 20:1; bevorzugt ist ein Verhältnis von 10 bis 20:1. Da aber bei einem Molverhältnis von 10:1 recht gute Ergebnisse erhalten werden, ist für eine technische Durchführung dieses Molverhältnis vorzuziehen, da hierdurch die Menge an umzuwälzendem Resorcin nicht unnötig groß ist.

Wäßriges Wasserstoffperoxid wird in üblichen Lösungen von 20–85 Gewichtsprozent Wasserstoffperoxid eingesetzt, bevorzugt zwischen 30–85 Gewichtsprozent.

Durch das erfindungsgemäße Verfahren ist es zum ersten Mal möglich geworden, direkt, d.h. ohne Verwendung eines Katalysators, aus Resorcin und wäßrigem Wasserstoffperoxid 1,2,3- und 1,2,4-Trihydroxybenzole herzustellen, und zwar mit sehr guten Ausbeuten, allein durch Verwendung höherer Temperaturen und eines geringen Wassergehalts vor Beginn der Reaktion. Ein solches Ergebnis war nach den Fehlschlägen gemäß dem Stand der Technik in den letzten hundert Jahren nicht vorauszusehen gewesen.

Die nachfolgenden Beispiele sollen die Erfindung weiter erläutern. Aus ihnen geht auch deutlich der Einfluß der Temperatur und des Wassergehaltes auf die Reaktion hervor.

Beispiel 1

110 g (1,0 mol) Resorcin werden auf 110°C erwärmt. Zu dieser Schmelze gibt man 2,52 g (0,05 mol) 70%iges Wasserstoffperoxid unter Rühren hinzu. Die Temperatur in der Reaktionslösung erhöht sich danach auf 133°C. Nach Abklingen der Exotherme wird nach 10 Minuten ein Wasserstoffperoxidumsatz von 97,8% bestimmt. Das Reaktionsgemisch enthält dann 5,5 g 1,2,4-Trihydroxybenzol und 0,85 g Pyrogallol, was einer Gesamtausbeute an Trihydroxybenzolen von 99,2%, bezogen auf umgesetztes Wasserstoffperoxid, entspricht.

Das Verhältnis von Hydroxyhydrochinon zu Pyrogallol beträgt 6,4 : 1.

Beispiel 2

55 g (0,5 mol) Resorcin werden auf 110°C erwärmt. Zu dieser Schmelze gibt man 2,43 g (0,05 mol) 70%iges Wasserstoffperoxid unter Rühren hinzu. Die Temperatur in der Reaktionslösung steigt hierbei auf 162°C an. Nach Abklingen der Exotherme wird nach 10 Minuten ein Wasserstoffperoxidumsatz von 99,8% bestimmt. Das Reaktionsgemisch enthält dann 4,8 g 1,2,4-Trihydroxybenzol und 0,95 g Pyrogallol, was einer Gesamtausbeute an Trihydroxybenzolen von 91,1%, bezogen auf umgesetztes Wasserstoffperoxid, entspricht.

Das Verhältnis von Hydroxyhydrochinon zu Pyrogallol beträgt 5,0 : 1.

Beispiel 3

55 g (0,5 mol) Resorcin werden auf 110°C erwärmt. Zu dieser gerührten Schmelze gibt man 2 g (0,05 mol) 85%iges Wasserstoffperoxid. Die Temperatur in der Reaktionslösung erhöht sich danach auf 135°C. Nach Abklingen der Exotherme wird nach 10 Minuten ein Wasserstoffperoxidumsatz von 98% bestimmt. Das Reaktionsgemisch enthält dann 4,65 g 1,2,4-Trihydroxybenzol und 1,1 g Pyrogallol, was einer Gesamtausbeute an Trihydroxybenzolen von 93%, bezogen auf umgesetztes Wasserstoffperoxid, entspricht.

Das Verhältnis von Hydroxyhydrochinon zu Pyrogallol beträgt 4,2 : 1.

Beispiel 4

55 g (0,5 mol) Resorcin werden auf 110°C erwärmt. Zu dieser gerührten Schmelze gibt man 5,7 g (0,05 mol) 30%iges Wasserstoffperoxid. Die Temperatur in der Reaktionslösung erhöht sich danach auf 137°C. Nach Abklingen der Exotherme wird nach 10 Minuten ein Wasserstoffperoxidumsatz von 99% bestimmt. Das Reaktionsgemisch enthält dann 4,68 g 1,2,4-Trihydroxybenzol und 1,35 g Pyrogallol, was einer Gesamtausbeute an Trihydroxybenzolen von 96,0%, bezogen auf umgesetztes Wasserstoffperoxid, entspricht.

Das Verhältnis von Hydroxyhydrochinon zu Pyrogallol beträgt 3,4 : 1.

Beispiel 5

55 g (0,5 mol) Resorcin werden auf 110°C in einer $N_2$-Atmosphäre erwärmt. Zu dieser gerührten Schmelze gibt man 4,86 g (0,1 mol) 70%iges Wasserstoffperoxid. Die Temperatur in der Reaktionslösung erhöht sich danach auf 175°C. Nach 10 Minuten wird ein Wasserstoffperoxidumsatz von 100% bestimmt. Das Reaktionsgemisch enthält dann 7,93 g 1,2,4-Trihydroxybenzol und 2,52 g Pyrogallol, was einer Gesamtausbeute an Trihydroxybenzolen von 83%, bezogen auf eingesetztes Wasserstoffperoxid, entspricht. Das Verhältnis von Hydroxyhydrochinon zu Pyrogallol beträgt 3,1 : 1.

Beispiel 6

55 g (0,5 mol) Resorcin werden auf 100°C erwärmt und mit 6 g Wasser versetzt. Zu dieser gesättigten Lösung gibt man 2,52 g (0,05 mol) 70%iges Wasserstoffperoxid unter starkem Rühren hinzu. Die Temperatur in der Reaktionslösung erhöht sich danach auf 112°C. Nach 20 Minuten wird ein Wasserstoffperoxidumsatz von 100% bestimmt. Das Reaktionsgemisch enthält dann 3,54 g 1,2,4-Trihydroxybenzol und 1,06 g Pyrogallol, was einer Gesamtausbeute an Trihydroxybenzolen von 70,3%, bezogen auf eingesetztes Wasserstoffperoxid, entspricht. Das Verhältnis von Hydroxyhydrochinon zu Pyrogallol beträgt 3,3 : 1.

Beispiel 7

55 g (0,5 mol) Resorcin werden auf 100°C erwärmt und mit 6 g Wasser versetzt. Zu dieser gesättigten Lösung von Resorcin in Wasser gibt man 5,67 g (0,05 mol) 30%iges Wasserstoffperoxid unter starkem Rühren hinzu. Die Temperatur in der Lösung erhöht sich danach auf 106°C. Nach 20 Minuten wird ein Wasserstoffperoxidumsatz von 100% bestimmt. Das Reaktionsgemisch enthält dann 2,7 g 1,2,4-Trihydroxybenzol und 1,55 g 1,2,3-Trihydroxybenzol, was einer Gesamtausbeute an Trihydroxybenzolen von 66,8%, bezogen auf eingesetztes Wasserstoffperoxid, entspricht. Das Verhältnis von Hydroxyhydrochinon zu Pyrogallol beträgt 1,74 : 1.

Beispiel 8 (zum Vergleich)

11 g (0,1 mol) Resorcin werden bei 30°C in 20 g Wasser aufgelöst und mit 4,86 g (0,1 mol) 70%igem Wasserstoffperoxid versetzt. Nach 3 Stunden Reaktionsdauer bei 30°C wird ein Wasserstoffperoxidumsatz von 60% bestimmt, wobei jedoch keine nachweisbare Menge an Trihydroxybenzolen gebildet worden ist.

Nach dieser Zeit wird die Temperatur auf 80°C erhöht. Nach einer Stunde Reaktionsdauer ist das Wasserstoffperoxid dann vollständig umgesetzt, wobei eine geringe Menge an Trihydroxybenzolen (<10%) gebildet worden ist.

Beispiel 9 (zum Vergleich)

11 g (0,1 mol) Resorcin werden bei 80°C in 20 g Wasser gelöst und mit 4,86 g (0,1 mol) 70%igem Wasserstoffperoxid versetzt. Während der Aufheizphase wurde bereits eine Braunfärbung beobachtet. Die Temperatur in der Reaktionslösung erhöht sich danach auf 90°C.

Nach 10 Minuten wird ein Wasserstoffperoxidumsatz von 100% bestimmt. Das Reaktionsgemisch enthält äußerst geringe Mengen (<10%) an Trihydroxybenzolen.

Beispiel 10

55 g (0,5 mol) Resorcin werden auf 80°C erwärmt und mit 8 g Wasser in Lösung aufgelöst. Zu dieser gesättigten Lösung gibt man 2,43 g (0,05 mol) 70%iges Wasserstoffperoxid unter starkem Rühren hinzu. Die Temperatur erhöht sich während der gesamten Umsetzung nicht.

Nach 20 bzw. 60 Minuten wird ein Wasserstoffperoxidumsatz von 92,9% bzw. 100% bestimmt. Das Reaktionsgemisch enthält dann 4,33 g 1,2,4-Trihydroxybenzol und 1,0 g 1,2,3-Trihydroxybenzol, was einer Gesamtausbeute an Trihydroxybenzolen von 84%, bezogen auf eingesetztes Wasserstoffperoxid, entspricht.

Beispiel 11

55 g (0,5 mol) Resorcin werden auf 60°C erwärmt und mit 13 g Wasser versetzt. Zu dieser gesättigten Lösung gibt man 2,43 g (0,05 mol) 70%iges Wasserstoffperoxid unter starkem Rühren hinzu. Die Temperatur in der Reaktionslösung erhöht sich nach der Zugabe nicht. Nach insgesamt 4 Stunden wird ein Wasserstoffperoxidumsatz von 98,2% bestimmt. Das Reaktionsgemisch enthält dann 2,26 g 1,2,4-Trihydroxybenzol und 0,6 g 1,2,3-Trihydroxybenzol, was einer Gesamtausbeute an Trihydroxybenzolen von 45%, bezogen auf eingesetztes Wasserstoffperoxid, entspricht.

**Patentansprüche**

1. Verfahren zur Herstellung von 1,2,3- und 1,2,4-Trihydroxybenzolen durch Hydroxylierung von Resorcin mit Wasserstoffperoxid, dadurch gekennzeichnet, daß man Resorcin bei Temperaturen von mindestens 60°C mit wäßrigem Wasserstoffperoxid in Abwesenheit eines Katalysators in Kontakt bringt, wobei die anfängliche Wassermenge des Gemisches aus Resorcin und Wasserstoffperoxid vor Beginn der Reaktion zwischen 0,1 bis 36 Gewichtsprozent, bezogen auf das Gemisch, liegt.

2. Verfahren nach Anspruch 1, dadurch gekennzeichnet, daß man bei mindestens 80°C Resorcin und Wasserstoffperoxid miteinander in Kontakt bringt.

3. Verfahren nach Anspruch 1 und 2, dadurch gekennzeichnet, daß man Resorcin mit Wasserstoffperoxid bei 110 bis 120°C in Kontakt bringt.

4. Verfahren nach Anspruch 1 bis 3, dadurch gekennzeichnet, daß man Resorcin in Form seiner Schmelze einsetzt.

5. Verfahren nach Anspruch 1 bis 4, dadurch gekennzeichnet, daß die Wassermengen vor Beginn der Reaktion zwischen 0,5 bis 15 Gewichtsprozent, bezogen auf das Gemisch aus Resorcin und Wasserstoffperoxid, liegen.

6. Verfahren nach Anspruch 1 bis 5, dadurch gekennzeichnet, daß die Wassermengen zwischen 0,8 bis 10 Gewichtsprozent, bevorzugt zwischen 0,8 bis 6 Gewichtsprozent, bezogen auf das Gemisch aus Resorcin und Wasserstoffperoxid, liegen.

**Claims**

1. Process for the preparation of 1,2,3- and 1,2,4-trihydroxybenzenes by hydroxylation of resorcinol with hydrogen peroxide, characterized in that resorcinol is brought into contact with aqueous hydrogen peroxide at temperatures of at least 60°C in the absence of a catalyst, the initial amount of water of the mixture consisting of resorcinol and hydrogen peroxide before the start of the reaction being between 0.1 and 36 percent by weight, relative to the mixture.

2. Process according to Claim 1, characterized in that resorcinol and hydrogen peroxide are brought into contact with one another at at least 80°C.

3. Process according to Claim 1 and 2, characterized in that resorcinol is brought into contact with hydrogen peroxide at 110 to 120°C.

4. Process according to Claim 1 to 3, characterized in that resorcinol is used in the form of its melt.

5. Process according to Claim 1 to 4, characterized in that the amounts of water before the start of the reaction are between 0.5 and 15 percent by weight, relative to the mixture consisting of resorcinol and hydrogen peroxide.

6. Process according to Claim 1 to 5, characterized in that the amounts of water are between 0.8 and 10 percent by weight, preferably between 0.8 and 6 percent by weight, relative to the mixture consisting of resorcinol and hydrogen peroxide.

## Revendications

1. Procédé pour la préparation de 1,2,3 et 1,2,4-trihydroxybenzènes par hydroxylation du résorcinol avec le peroxyde d'hydrogène, caractérisé en ce que l'on met le résorcinol en contact avec du peroxyde d'hydrogène en solution aqueuse, à des températures d'au moins 60°C, en absence d'un catalyseur, la teneur initiale en eau du mélange de résorcinol et peroxyde d'hydrogène étant comprise, avant la réaction, entre 0,1 et 36% en poids par rapport au mélange.

2. Procédé selon la revendication 1, caractérisé en ce que l'on met en contact le résorcinol et le peroxyde d'hydrogène l'un avec l'autre à au moins 80°C.

3. Procédé selon la revendication 1 ou 2, caractérisé en ce que l'on met en contact le résorcinol et le proxyde d'hydrogène à 110–120°C.

4. Procédé selon l'une quelconque des revendications 1 à 3, caractérisé en ce que l'on utilise le résorcinol sous forme d'une masse fondue.

5. Procédé selon l'une quelconque des revendications 1 à 4, caractérisé en ce qu'avant la réaction, les quantités d'eau sont comprises entre 0,5 et 15% en poids, par rapport au mélange de résorcinol et peroxyde d'hydrogène.

6. Procédé selon l'une quelconque des revendications 1 à 5, caractérisé en ce que les quantités d'eau sont comprises entre 0,8 et 10% en poids, de préférence entre 0,8 et 6% en poids, par rapport au mélange de résorcinol et peroxyde d'hydrogène.